# EUROPÄISCHE PATENTSCHRIFT

(11) **EP 2 538 949 B1**
(45) Veröffentlichungstag und Bekanntmachung des Hinweises auf die Patenterteilung: **21.01.2015**
(21) Anmeldenummer: 11706800.7
(22) Anmeldetag: 22.02.2011
(51) Int. Cl.: A61K 33/00, A61K 31/80, A61P 17/00, A61P 29/00, A61P 37/00, A61K 31/765, C04B 35/624, C04B 35/622, C04B 35/626

(54) **SILICIUMHALTIGES, BIOLOGISCH DEGRADIERBARES MATERIAL ZUR ANTI-INFLAMMATORISCHEN THERAPIE**
SILICON-CONTAINING BIODEGRADABLE MATERIAL FOR ANTI-INFLAMMATORY THERAPY
MATÉRIAU BIOLOGIQUEMENT DÉGRADABLE CONTENANT DU SILICIUM DESTINÉ À UNE THÉRAPIE ANTI-INFLAMMATOIRE

(30) Priorität: 24.02.2010 DE 102010008982
(43) Veröffentlichungstag der Anmeldung: 02.01.2013
(73) Patentinhaber: Bayer Innovation GmbH, 51373 Leverkusen (DE)
(72) Erfinder: BAECKER, Iwer, 40219 Düsseldorf (DE); SUSCHEK, Christoph, 40764 Langenfeld (DE)
(74) Vertreter: Kutzenberger Wolff & Partner
(86) Internationale Anmeldenummer: PCT/EP2011/052560
(87) Internationale Veröffentlichungsnummer: WO 2011/104214

(56) Entgegenhaltungen:
- EP-A1- 0 621 038
- WO-A2-2009/052090
- DE-A1-102007 061 873
- US-B1- 6 211 393
- ZHANG HANZHE ET AL: "Vascular endothelial growth factor promotes brain tissue regeneration with a novel biomaterial polydimethylsiloxane-tetraethoxysilane", BRAIN RESEARCH, Bd. 1132, Nr. 1, Februar 2007 (2007-02), Seiten 29-35, XP002631231, ISSN: 0006-8993
- KANG Y M ET AL: "Evaluations of osteogenic and osteoconductive properties of a non-woven silica gel fabric made by the electrospinning method", ACTA BIOMATERIALIA, ELSEVIER, AMSTERDAM, NL, Bd. 5, Nr. 1, 1. Januar 2009 (2009-01-01), Seiten 462-469, XP025804562, ISSN: 1742-7061, DOI: DOI:10.1016/J.ACTBIO.2008.07.004 [gefunden am 2008-07-17]

## Beschreibung

Die vorliegende Erfindung betrifft ein siliciumhaltiges, biologisch degradierbares Material zur Prophylaxe und/oder zur Behandlung von Krankheiten, die mit einer erhöhten Interleukin-1β und/oder Interleukin-6 und/oder Interleukin-8 Aktivität einhergehen bzw. die durch Erniedrigung einer solchen bzw. solcher Cytokinaktivität behandelt werden können.

Silicium ist ein Spurenelement, dass in gebundener silikatischer Form für den Menschen wichtig ist. Silicium ist ein Baustein derjenigen Eiweiße, die für die Festigkeit und Elastizität der Gewebe verantwortlich sind. Dabei ist es auch in Bindegewebe, Knochen, Haut, Haare, Nägel und Blutgefässe eingebaut. Zudem stärkt Silicium das Abwehrsystem des Körpers, das sogenannte Immunsystem, und fördert die Wundheilung. Ein Mangel an Silicium hat Wachstumsstörungen, einen Verlust der Knochenstabilität mit erhöhtem Risiko für Osteoporose sowie vorzeitigen Haarausfall, brüchige Nägel und Veränderungen der Haut zur Folge. Mögliche Veränderungen der Haut sind vermehrte Faltenbildung, Trockenheit, Schuppung, vermehrte Hornbildung, Juckreiz, Verdickung und schmerzhafte, spaltförmige Einrisse der Haut wegen herabgesetzter Elastizität. Zudem wird das Abwehrsystem des Körpers, das sogenannte Immunsystem, durch den Siliciummangel geschwächt und es besteht eine gesteigerte Anfälligkeit für Infekte.

US2006/0178268A1 beschreibt eine wässrige Lösung bestehend aus nicht-kolloidaler Kieselsäure und Borsäure zur Behandlung von Knochen-, Knorpel-, Haut-, Arterien-, Bindegewebe-, Gelenk-, Haar-, Nägel-, Hauterkrankungen sowie Osteoporose, rheumatischen Erkrankungen, Arteriosklerose, Arthritis, kardiovaskuläre Erkrankungen, allergische Erkrankungen und degenerative Erkrankungen.

US2006/0099276A1 offenbart eine Methode zur Herstellung eines Kieselsäure-Derivates durch Hydrolyse einer Silikonverbindung zu Oligomeren unter gleichzeitiger Anwesenheit einer quartären Ammonium Verbindung, einer Aminosäure oder einer Aminosäurequelle bzw. Kombinationen davon. Das Kieselsäure-Extrudat kann als Pharmazeutika zur Behandlung von Infektionen, Nägel-, Haar-, Haut-, Zahn-, Kollagen-, Bindegewebe-, Knochenerkrankungen, Osteopenie, zur Zellbildung für degenerative (Alterungs-)prozesse eingesetzt werden.

US6,335,457B1 offenbart eine Festkörper bei welchem Kieselsäure komplexiert mit einem Polypeptid vorliegt. Dieses Patent offenbart auch therapeutisch verwendbare Mischungen die diesen Festkörper beinhalten.

WO2009/018356A1 betrifft eine Mischung beinhaltend eine Natriumphosphat-Verbindung, eine Ammoniumverbindung und ein Silikat zur Prophylaxe oder Behandlung von Krankheiten wie Prostatakrebs, Kolorektaler Krebs, Lungenkrebs, Brustkrebs, Leberkrebs, Neuronaler Krebs, Knochenkrebs, HIV Syndrom, Rheumatische Arthritis, Multiple Sklerose, Epstein Barr Virus, Fibromyalgie, Chronisches Müdigkeitssyndrom, Diabetes, Bechets Syndrom, Reizdarmsyndrom, Crohn's Krankheit, Dekubitus, tropische Geschwüre, durch Strahlen- bzw. Chemotherapie geschwächtes Immunsystem, Hämatome bzw. Kombinationen davon.

WO2009/052090A2 beschreibt eine Methode zur Behandlung von inflammatorischen Krankheiten, Autoimmunerkrankungen, bakteriellen oder viralen Infektionen und Krebs durch Verwendung einer Zusammensetzung die Silikat enthält.

US2003/0018011A1 betrifft eine pharmazeutische Zusammensetzung mit einer Fettsäure und einem wasserlöslichen Silikat-Polymer als anti-allergisches oder als anti-inflammatorisches Agens.

US5,534,509 betrifft eine pharmazeutische Zusammensetzung enthaltend ein wasserlösliches Silikat-Polymer als aktivem Agens mit einem Saccharid oder Zuckeralkohol als inerter Trägersubstanz für die Behandlung von Allergien, Entzündungen, Schmerz oder zur Verbesserung der peripheren Blutzirkulation oder Parästhesie.

DE19609551C1 beschreibt die Herstellung von biologisch resorbierbaren (Endlos)Fasern auf der Basis von Polyhydroxykieselsäureethylester. Die Fasern werden als Verstärkungsfasern für biologisch degradierbare und/oder biologisch resorbierbare (Implantat) Werkstoffe eingesetzt. Die Fasern können auch zur Herstellung von biologisch degradierbaren Verbundmaterialien eingesetzt werden.

WO01/42428A1 beschreibt ein Verfahren zur Herstellung eines Hautimplantats, wobei man Hautzellen auf die Oberfläche einer Nährlösung aufbringt und diese mit Hilfe eines Flächenelements bestehend aus der in der DE19609551C1 beschriebenen Fasern wachsen lässt.

EP1262542A2 betrifft ein Verfahren zur *in-vitro*-Herstellung von Zellen, Geweben und Organen, wobei eine Fasermatrix als Zellstützsubstanz und/oder Leitstruktur gemäß der DE19609551C1 eingesetzt wird.

WO2006/069567A2 betrifft einen Multilagen-Verband bei dem in einer Lage auch eine Fasermatrix gemäß DE19609551C1 eingesetzt wird. Der Multilagenverband kann für die Behandlung von Wunddefekten, wie chronische diabetisch-neuropathischer Ulcus, Ulcus cruris, Dekubituswunden, sekundär heilende infizierte Wunden, reizlose, primär heilende Wunden, wie insbesondere ablative Riß- oder Schürfwunden eingesetzt werden.

WO2008/086970A1, WO2008148384A1, PCT/EP2008/010412 und PCT/EP2009/004806 beschreiben unter anderem die Herstellung von weiteren erfindungsgemäß verwendbaren Polyhydroxykieselsäureethylester-Verbindungen. Die Verbindungen werden allgemein für den Einsatz als biologisch resorbierbare Materialien in der Humanmedizin, der Medizintechnik, der Filtertechnik, der Biotechnologie oder der Dämmstoffindustrie beschrieben. Es wird auch erwähnt, dass die Materialien vorteilhaft im Bereich der Wundbehandlung und Wundheilung eingesetzt werden können. Fasern können beispielsweise als chirurgisches Nahtmaterial oder als Verstärkungsfasern eingesetzt werden. Vliese können bei der Versorgung von oberflächlichen Wunden, bei Filtration von Körperflüssigkeiten (z.B. Blut) oder im Bereich der Bioreaktoren als Anzuchthilfe verwendet werden.

US 6211393 offenbart anti-entzündliche Eigenschaften bestimmter Silicium-haltiger Verbindungen.

Im Stand der Technik ist nicht offenbart, dass man die oben genannten biologisch degradierbaren Polyhydroxykieselsäureethylester-Verbindungen (z.B. in Form einer Faser oder eines Vlieses) zur Prophylaxe und/oder die zur Behandlung von Krankheiten, die mit einer erhöhten Interleukin-1β und/oder Interleukin-6 und/oder Interleukin-8 Aktivität einhergehen bzw. die durch Erniedrigung einer solchen bzw. solcher Cytokinaktivität behandelt werden können, verwenden kann. Zwar wird in den oben aufgeführten Dokumenten die Verwendung der Polyhydroxykieselsäureethylester-Verbindungen für die Wundbehandlung und Wundheilung beschrieben und es ist bekannt, dass die Wundheilung mit pro- bzw. antiinflammatorischen Prozessen assoziiert ist, der Stand der Technik beschreibt jedoch nicht, dass man die oben genannten biologisch degradierbaren Polyhydroxykieselsäureethylester-Verbindungen generell prophylaktisch und/oder therapeutisch für insbesondere inflammatorische und/oder autoimmunologische Krankheiten einsetzen kann.

Gegenstand der vorliegenden Erfindung ist deshalb ein Siliciumhaltiges, biologisch degradierbares Material zur Prophylaxe und/oder zur Behandlung von Krankheiten, die mit einer erhöhten Interleukin-1β und/oder Interleukin-6 und/oder Interleukin-8 Aktivität einhergehen bzw. und/oder Krankheiten bei denen eine Reduktion der Aktivität von Interleukin-1β und/oder Interleukin-6 und/oder Interleukin-8 förderlich für den Heilungsprozess ist, wobei das Siliciumhaltige, biologisch degradierbare Material eine Polyhydroxykieselsäureethylester-Verbindung ist, mit der Maßgabe das Wunddefekte, wie chronische diabetisch-neuropathische Ulcus, Ulcus cruris, Dekubituswunden, sekundär heilende infizierte Wunden, reizlose, primär heilende Wunden, wie insbesondere ablative Riß- oder Schürfwunden ausgenommen sind. Von der Erfindung umfasst ist auch die Verwendung einer Siliciumhaltigen, biologisch degradierbaren Polyhydroxykieselsäureethylester-Verbindung zur Herstellung eines Medikaments zur Prophylaxe und/oder zur Behandlung von Krankheiten, die mit einer erhöhten Interleukin-1β und/oder Interleukin-6 und/oder Interleukin 8 Aktivität einhergehen und/oder Krankheiten bei denen eine Reduktion der Aktivität von Interleukin-1β und/oder Interleukin-6 und/oder Interleukin-8 förderlich für den Heilungsprozess ist, mit der Maßgabe das Wunddefekte, wie chronische diabetisch-neuropathische Ulcus, Ulcus cruris, Dekubituswunden, sekundär heilende infizierte Wunden, reizlose, primär heilende Wunden, wie insbesondere ablative Riß- oder Schürfwunden ausgenommen sind.

Von der Erfindung ausgeklammert sind diejenigen Anwendungen des erfindungsgemäßen Materials, welche in den folgenden Patentschriften beschrieben sind DE19609551C1, WO01/42428A1, EP1262542A2, WO2006/069567A2, WO2008/086970A1, WO2008148384A1, PCT/EP2008/010412 und PCT/EP2009/004806 und die mit der vorliegenden Erfindung in Zusammenhang gebracht werden. Die Verwendung eines Polyhydroxykieselsäureethylesterfaservlieses als Bestandteil eines Multilagenverbandes ist zur Behandlung von Wunddefekten, wie chronische diabetisch-neuropathische Ulcus, Ulcus cruris, Dekubituswunden, sekundär heilende infizierte Wunden, reizlose, primär heilende Wunden, wie insbesondere ablative Riß- oder Schürfwunden in der WO2006/069567A2 beschrieben. Die EP1262542A2 beschreibt verschiedenste Tissue-Engineering Anwengungen von erfindungsgemäßen Polyhydroxykieselsäureethylester-Verbindungen. Der Begriff "Tissue-Engineering Anwendungen" gemäß vorliegender Erfindung orientiert sich an dem in der EP1262542A2 beschriebenen Produkten, Verfahren und Anwendungen. Von der Erfindung ausgenommen sind deshalb die in der EP1262542A2 diskutierten Tissue-Engineering Anwendungen des erfindungsgemäßen siliciumhaltigen und biologisch degradierbaren Materials, sofern diese mit dem Gegenstand der vorliegenden Erfindung in Zusammenhang gebracht werden sollten.

Der Begriff ""Polyhydroxykieselsäureethylester-Verbindung" beschreibt erfindungsgemäß alle Verbindungen der allgemeinen Formel H[OSi₈O₁₂(OH)ₓ(OC₂H₅)₆₋ₓ]ₙOH, bei der x für 2 bis 5 steht und n > 1 ist (Polymer).

Das erfindungsgegenständliche siliciumhaltige, biologisch degradierbare Material liegt vorzugsweise als Material in Form einer Faser, einer Fasermatrix, als Pulver, als Monolith und/oder als Beschichtung vor. Ein solches siliciumhaltiges, biologisch degradierbares Material kann erfindungsgemäß wie nachfolgend beschrieben hergestellt werden:
a) mindestens einer Hydrolyse-Kondensationsreaktion von Tetraethoxysilan,
b) Eindampfen zur Erzeugung einer einphasigen Lösung vorzugsweise bei einer gleichzeitigen schonenden Durchmischung des Reaktionssystems,
c) Abkühlung der einphasigen Lösung und
d) Reifung zur Erzeugung eines Kieselsol-Materials,
e) Ziehen von Fäden von dem Kieselsol-Material zur Generierung einer Faser bzw. einer Fasermatrix und/oder Trocknen und insbesondere Sprüh- oder Gefriertrocknen des Kieselsol-Materials zur Generierung eines Pulver und ggfs. Lösen des Pulvers in einem Lösungsmittel zur Generierung einer flüssigen Formulierung und/oder Beschichten eines mit dem siliciumhaltigen, biologisch degradierbaren Material zu beschichtendem Gegenstandes mit dem Kieselsol-Material, und/oder Gießen des Kieselsol-Materials in eine Form zur Generierung eines Monoliths.

Erfindungsgemäß bevorzugt liegt das siliciumhaltige, biologisch degradierbaren Material der Erfindung als Faser, Fasermatrix (Vlies), als Pulver, als flüssige Formulierung und/oder als Beschichtung vor.

In einer weiteren Ausführungsform der Erfindung wird das gegenständliche siliciumhaltige, biologisch degradierbare Material wie oben beschrieben hergestellt, wobei das Tetraethoxysilan in Schritt a) bei einem anfänglichen pH-Wert von 0 bis ≤ 7, gegebenenfalls in Gegenwart eines wasserlöslichen Lösungsmittels, vorzugsweise Ethanol, bei einer Temperatur von 0°C bis 80°C sauer katalysiert wird und in Schritt b) das Eindampfen bis zu einer einphasigen Lösung mit einer Viskosität im Bereich von 0,5 bis 2 Pa • s bei einer (Scherrate von 10 s⁻¹ bei 4 °C durchgeführt wird.

In einer weiteren Ausführungsform der Erfindung wird das siliciumhaltige, biologisch degradierbare Material wie oben beschrieben hergestellt, wobei die saure Katalyse in Schritt a) mit salpetersaurem H₂O in einem molaren Verhältnis zur Si-Verbindung im Bereich 1:1,7 bis 1:1,9, bevorzugt im Bereich von 1:1,7 bis 1:1,8 durchgeführt wird. Die Hydrolyse-Kondensationsreaktion in Schritt a) erfolgt vorzugsweise bei einer Temperatur von 20 bis 60°C, bevorzugt 20 bis 50°C über einen Zeitraum von mindestens einer Stunde. Bevorzugt läuft die Hydrolyse-Kondensationsreaktion in Schritt a) über einen Zeitraum von mehreren Stunden wie beispielsweise 8 h oder 16 h ab. Diese Reaktion kann aber auch in einem Zeitraum von 4 Wochen durchgeführt werden. Schritt (b) wird in einer bevorzugten Ausführungsform der Erfindung in einem geschlossenen Apparat, in dem eine Durchmischung möglich ist (vorzugsweise Rotationsverdampfer oder Rührkessel) bei gleichzeitiger Entfernung des Lösungsmittels (Wasser, Ethanol) durch Eindampfen bei einem Druck von 1 bis 1013 mbar, bevorzugt bei einem Druck von < 600 mbar, optional mit kontinuierlicher Zufuhr eines chemisch inerten Schleppgases zur Partialdruckerniedrigung der verdampfenden Komponenten von 1 - 8m³/h (vorzugsweise bei 2,5 bis 4,5 m³/h), einer Reaktionstemperatur von 30°C bis 90°C, vorzugsweise 60 bis 75°C, noch bevorzugter bei 60 bis 70°C und vorzugsweise unter schonender Durchmischung des Reaktionssystems bis 80U/min (vorzugsweise bei 20U/min bis 80U/min) bis zu einer Viskosität des Gemisches auf 0,5 bis 30 Pa • s bei einer Scherrate von 10 s⁻¹ bei 4 °C, vorzugsweise 0,5 bis 2 Pa • s bei einer Scherrate von 10 s⁻¹ bei 4 °C, besonders bevorzugt ca. 1 Pa • s (Messung bei 4°C, Scherrate 10 s⁻¹), durchgeführt. In einer weiteren Ausführungsform der Erfindung wird das siliciumhaltige, biologisch degradierbare Material in Schritt c) auf vorzugsweise 2 °C bis 4 °C, abgekühlt. Bei diesen tiefen Temperatur erfolgt vorzugsweise auch die Reifung (Schritt d). Die Reifung kann mehrere Stunden bzw. Tage bis etwa 3 bis 4 Wochen benötigen. Der Reifeprozess in Schritt d) wird vorzugsweise bis zu einer Viskosität des Sols von 30 bis 100 Pa • s bei einer Scherrate von 10 s⁻¹ bei 4 °C und einem Verlustfaktor von 2 bis 5 (bei 4°C, 10 1/s, 1% Deformation) durchgeführt.

Das Ziehen von Fäden von dem Kieselsol-Material in Schritt e) wird vorzugsweise über einen Spinnprozess durchgeführt. Ein solcher Spinnprozessschritt kann unter üblichen Bedingungen durchgeführt werden, wie zum Beispiel in DE 196 09 551 C1 und DE 10 2004 063 599 A1 beschrieben.

Das Trocknen des Kieselsol-Materials zur Generierung von Pulver wird vorzugsweise durch ein Sprüh- oder Gefriertrocknen durchgeführt. Ein Pulver kann auch erhalten werden, indem Monolithe bzw. auch erfindungsgemäße Fasern zerkleinert und gemahlen werden. Zur Generierung einer flüssigen Formulierung wird das Pulver in einem Lösungsmittel gelöst. Geeignete Lösungsmittel können je nach Anwendung (z.B. Injektionslösung bzw. Suspensionen) wässrig oder ölig sein.

Das Beschichten eines mit dem siliciumhaltigen, biologisch degradierbaren Material zu beschichtendem Gegenstandes mit dem Kieselsol-Material erfolgt vorzugsweise durch Tauchen des zu beschichtenden Körpers in das Kieselsol, durch Begießen oder durch Aufschleudern oder Sprayen des Kieselsols.

Das Kieselsol-Materials gemäß Schritt d) kann auch - zur Generierung eines Monoliths - in eine Form gegossen und anschließend getrocknet werden.

Weitere konkretisierenden Angaben bezüglich der Herstellung der erfindungsgegenständlichen siliciumhaltigen, biologisch degradierbaren Material sind der DE19609551C1, WO01/42428A1, EP1262542A2, WO2006/069567A2, WO2008/086970A1, WO2008148384A1, PCT/EP2008/010412 und der PCT/EP2009/004806 zu entnehmen.

Im Sinne der vorliegenden Erfindungen bezeichnet der Ausdruck "biologisch degradierbar" die Eigenschaft der erfindungsgemässen Polyhydroxykieselsäureethylester-Verbindung abgebaut zu werden, wenn das Material Bedingungen ausgesetzt wird, die typisch sind für die diejenigen, die bei einer Geweberegeneration (beispielsweise einer Wunde) vorliegen. "Biologisch degradierbar" bzw. "biodegradierbar" im Sinne der Erfindung ist die erfindungsgemäße Polyhydroxykieselsäureethylester-Verbindung insbesondere dann, wenn sie sich nach 48 Stunden, vorzugsweise 36 Stunden und besonders bevorzugt nach 24 Stunden in einer 0,05 M Tris pH 7,4 Pufferlösung (Fluka 93371) thermostatisiert bei 37°C vollständig löst.

Zytokine und Chemokine regulieren Entzündungen und Reaktionen auf Infektionen, Verletzungen und Krebs. Während einige Zytokine Reaktionen auf körperfremdes wie körpereigenes Material verstärken, reduzieren andere Zytokine Entzündungen und fördern so die Heilung. Die Expression und Produktion nahezu aller pro-inflammatorischen Zytokine wird im Rahmen von Entzündungsreaktionen über Signalwege gesteuert, die durch den Transkriptionsfaktor NFκB reguliert werden. NFκB ist somit das zentrale regulatorische Molekül auch in der Induktion von Interleukin-1β, Interleukin-6 und Interleukin-8. Interleukin-1β, Interleukin-6 und Interleukin-8 sind entzündungsverstärkende bzw. entzündungsinitiierende Zytokine, die mit Fieber, Entzündungen, Zerstörung des Gewebes und in einigen Fällen Schock und Tod einhergehen. Die Verringerung der biologischen Aktivität von IL-1β und/oder IL-6 und/oder IL-8 ist deshalb Ziel verschiedener therapeutischer Maßnahmen und wird erfolgreich angewendet bei einer Vielzahl von Entzündungskrankheiten.

Der Begriff "Krankheiten, die mit einem erhöhten Interleukin-1β und/oder Interleukin-6 und/oder Interleukin-8 Aktivität einhergehen und/oder Krankheiten bei denen eine Reduktion der Aktivität von Interleukin-1β und/oder Interleukin-6 und/oder Interleukin-8 förderlich für den Heilungsprozess ist" beschreibt Krankheiten umfassend:

Autoimmunerkrankungen, Allergien, Enteritis, Kolitis, Gastritis, Arthritis, Myokarditis, Dermatitis, Otitis, Pneumonitis, Schocklunge, Blutgerinnungsstörungen, entzündliche Knochen- und Gelenkerkrankungen, Rheumatoide Arthritis, Sepsis, septischer Schock, Folgeerscheinungen nach Transplantationen, akute und chronische Entzündungen, entzündliche Darmerkrankung (Bowel disease), Graft-versus-Host-Krankheit, Schock, Schlaganfall, akutes respiratorisches Syndrom (ARDS), Psoriasis, Restenose, AIDS, Kachexie, Schädel-Hirn-Trauma. Allergie, parasitäre Infektion, allergische Rhinitis, allergisches Asthma, atopische Dermatitis, Multiple Sklerose, Systemischer Lupus erythematodes, Graft-versus-Host-Krankheit, Transplantatabstoßung, Asthma und chronisch obstruktiver Lungenerkrankung, chronische Gastritis, akute und chronische Entzündungen der Haut, Psoriasis, Allergien der Haut, parasitäre Infektion der Haut, atopische Dermatitis insbesondere Neurodermitis, Dermatomyositis, Pemphigus vulgaris und/oder andere lokale und systemische Infektionen und/oder akute und chronische Entzündungssituationen.

Zu den Autoimmunkrankheiten zählen insbesondere folgende Krankheiten: Alopecia areata, Antiphospholipid-Syndrom, Amyotrophe Lateralsklerose, Autoimmune Polyendocrinopathy-Candidiasis-Ectodermal Dystrophy (Polyendokrine Autoimmunerkrankung), Arteriitis temporalis, Atherosklerose, Autoimmunenteropathie, CREST-Syndrom, Crohns-Krankheit (Morbus Crohn), Dermatomyositis, Dermatitis herpetiformis Duhring, Diabetes mellitus type 1, Epidermolysis bullosa acquisita, Fibromyalgie, chronische autoimmune Gastritis, Goodpasture-Syndrom, Guillain-Barré-Syndrom, Hashimoto-Thyreoiditis, PANDAS, Hyperthyreose, idiopathische thrombozytopenische Purpura, Bindegewebserkrankung, Juvenile rheumatoide Arthritis, Koronare Herz-Erkrankungen, Kraniomandibuläre Dysfunktion, Kryoglobulinämie, Kälteagglutininkrankheit, Lichen sclerosus, Lineare IgA Dermatose, Lyme-Arthritis, Lupus erythematodes, Mischkollagenose, Morbus Addison, Morbus Basedow, Myasthenia gravis, Narkolepsie, Pemphigus vulgaris, perniziöse Anämie (Morbus Biermer), Morbus Werlhof, Myasthenia gravis, Polymyositis, primär biliäre Zirrhose, Polymyalgia rheumatica, rheumatoide Arthritis, Sjögren Syndrom, Sklerodermie, Stiff-Man-Syndrom, Sympathische Ophthalmie, Riesenzellarteriitis, Colitis ulcerosa, Vaskulitis, Wegener-Granulomatose, Morbus Bechterew, Autoimmunhepatitis, bullösen Pemphigoid, Pemphigus seborrhoicus, Polyangiitis, Polyneuropathie, Reiter Syndrom, Rheumatisches Fieber, Churg-Strauss-Syndrom, Sinusitis, Sarkoidose, Takayasu Arteriitis, Toxoplasmose, Glomerulonephritis, Hashimoto-Thyreoiditis, Morbus Adamantiades-Behçet, Rezidivierende Polychondritis, Relapsing Polychondritis, Panchondritis, Systematisierte Chondromalazie, Polychondritis atropicans, polyendokrine Autoimmuninsuffizienz, Purpura Schönlein-Henoch *(Purpura anaphylactoides*, *Vasculitis allergica)*, Vitiligo, Zöliakie, autoimmune Nephritiden und Vaskulitiden und/oder autoimmunbedingte Leukämien.

Krankheiten, die mit der erfindungsgemäßen Verbindung bevorzugt behandelt werden sind akute und chronische Entzündungen der Haut, Psoriasis, Allergien der Haut, parasitäre Infektion der Haut, atopische Dermatitis insbesondere Neurodermitis, Dermatomyositis und/oder Pemphigus vulgaris.

Die geeignete Dosierung der erfindungsgemäß verwendeten Polyhydroxykieselsäureethylester-Verbindung ist generell insgesamt zwischen 0,001 bis zu 100 mg/kg Körpergewicht pro Tag und verabreicht in ein- bzw. mehrmaligen Dosierungen. Vorzugsweise wird eine Dosierung zwischen 0,01 und 25 mg/kg, noch bevorzugter 0,1 bis 5 mg/kg pro Tag eingesetzt. Die bioabbaubaren Eigenschaften der Polyhydroxykieselsäureethylester-Verbindungen ermöglichen es aber auch, dass die Verbindungen in höherer Dosierungen appliziert werden können und beispielsweise innerhalb des Körpers z.B. subkutan als Depot in Form eines Monolithen über längere Zeit degradieren.

Das erfindungsgemäßen Material bzw. eine Vorstufe davon (wie beispielsweise das oben in Schritt d) beschriebene Kieselsol-Material) kann mit den in der Galenik gebräuchlichen Trägersubstanzen, Füllstoffen, Zerfallsbeeinflussern, Bindemitteln, Gleitmitteln, Absorptionsmitteln, Verdünnungsmitteln, Geschmackskorrigentien, Färbemitteln usw. verarbeitet und in die gewünschte Applikationsform überführt. Dabei ist auf Remington's Pharmaceutical Science, 15th ed. Mack Publishing Company, East Pennsylvania (1980) hinzuweisen.

Verabreicht kann das erfindungsgemäßen Materials in einer geeigneten Applikationsform oral, mucosal (wie beispielsweise sublingual, buccal, rectal, nasal oder vaginal), parenteral (wie beispielsweise subkutan, intramuskulär, mittels Bolusinjektion, intraarterial, intravenös), transdermal oder lokal (wie beispielsweise direkte Applikation auf der Haut oder topische Applikation auf ein exponiertes Organ oder eine Wunde).

Für die orale Applikation kommen insbesondere Tablette, Dragees, Filmtabletten, Kapseln, Pillen, Pulver, Granulate, Pastillen, Suspensionen, Emulsionen oder Lösungen in Frage.

Tabletten, Dragees, Kapseln etc. können beispielsweise wie oben beschrieben durch Gießen des in Schritt d) erhaltenen Kieselsol-Materials in eine tabletten- bzw. kapselartige Form zur Generierung eines Monoliths erhalten werden kann. Die Tabletten und Kapseln können aber auch über das oben beschriebene erfindungsgemäße Material in Form eines Pulvers nach üblichen Verfahren hergestellt werden. Das erfindungsgemäßen Material bzw. eine Vorstufe davon kann mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinyläcetat versetzt werden. Tabletten können auch aus mehreren Schichten bestehen. Die erfindungsgegenständlichen Materialien enthaltenden Kapseln können beispielsweise hergestellt werden, indem man die erfindungsgemäßen Materialien bzw. eine Vorstufe davon mit einem inerten Träger wie Milchzucker oder Sorbit mischt und in Gelatinekapseln einkapselt. Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Für die parenterale Applikation sind Injektions- und Infusionszubereitungen möglich. Für die intraartikuläre Injektion können entsprechend zubereitete Kristallsuspensionen verwendet werden. Für die intramuskuläre Injektion können flüssige Formulierungen wie wässrige und ölige Injektionslösungen oder Suspensionen und entsprechende Depotpräparationen Verwendung finden. Für die rektale Applikation können die erfindungsgemäßen Materialien in Form von Suppositorien, Kapseln, Lösungen (z.B. in Form von Klysmen) und Salben sowohl zur systemischen als auch zur lokalen Therapie verwendet werden. Weiterhin seien als Zubereitung auch Mittel zur vaginalen Anwendung genannt. Flüssige Formulierungen wie Injektionslösungen bzw. Suspensionen können beispielsweise dadurch erhalten werden, indem das oben beschriebene erfindungsgemäße Material in Form eines Pulvers mit geeigneten wässrigen bzw. öligen Lösungsmitteln versetzt wird. Andere Herstellungsarten sind dem Fachmann bekannt. Lösungen oder Suspensionen des erfindungsgemäßen Materials können zusätzlich geschmacksverbessernde Mittel wie Saccharin, Cyclamat oder Zucker sowie z.B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Geeignete Suppositorien lassen sich beispielsweise durch Vermischen mit dafür vorgesehenen Trägermitteln wie Neutralfetten oder Polyethylenglykol bzw. deren Derivaten herstellen.

Für die transdermale Applikation sind Pflaster bzw. für die topische Auftragung Formulierungen in Gelen, Salben, Fettsalben, Cremes, Pasten, Puder, Milch und Tinkturen möglich. Wundpflaster bestehen vorzugsweise aus Fasern bzw. einer Fasermatrix (Vlies) aus den erfindungsgemäßen Materialien so wie im Stand der Technik beschrieben.

In einer weiteren Ausführungsform der Erfindung kann das erfindungsgemäße Material bzw. eine Vorstufe davon mit einem Beschichtungsverfahren, zum Beispiel durch Tauchen eines zu beschichtenden Gegenstandes bzw. Körpers in das oben in Schritt d) beschriebenen Kieselsol-Materials, durch Begießen oder durch Aufschleudern oder Sprayen eines solchen Kieselsol-Materials beschichtet werden. Beispielsweise kann das erfindungsgemäße Kieselsol-Material auf Pflaster oder Verbände beschichtet werden.

Die oben genannten Applikationsformen können auch weitere pharmazeutische Wirkstoffe (jedoch nicht als Teil der Polyhydroxykieselsäureethylester-Verbindung) enthalten, die während dem Herstellprozess dazugefügt werden können.

### Beispiele

### 1. Herstellung einer erfindungsgemäßen Fasermatrix aus Polyhydroxykieselsäureethylester

Als Edukt für die Hydrolyse-Kondensationsreaktion wurden 1124,98 g TEOS (Tetraethoxysilan) in einem Rührkessel vorgelegt. 313,60 g EtOH als Lösungsmittel wurden zugegeben. Das Gemisch wird gerührt. Separat wurde 1 n HNO₃ (55,62 g) mit H₂O (120,76 g) verdünnt und dem TEOS-Ethanol-Gemisch hinzugefügt. Das Gemisch wurde 18 Stunden gerührt.

Das durch diesen Schritt erhaltene Gemisch wurde nachfolgend bei Temperaturen von 62°C unter Zufuhr eines Schleppstroms und Rühren (60 U/min) bis zu einer dynamischen Viskosität (Scherrate 10 s-1 bei 4°C) von 1 Pa • s eingedampft.

Die Lösung wurde anschließend in einem geschlossenen Polypropylen-Reifebecher ruhend und aufrecht bei einer Temperatur von 4°C bis zu einer dynamischen Viskosität von ca. 55 Pa • s (Scherrate 10 s-1 bei 4°C) und einem Verlustfaktor von 3,0 gereift.

Das durch die Reifung entstandene Sol wurde dann zur Faser versponnen. Die Herstellung der Fasern erfolgte in einer üblichen Spinnanlage. Dazu wurde die Spinnmasse in einen auf -15°C gekühlten Druckzylinder gefüllt. Die Spinnmasse wurde mit Druck durch die Düsen gepresst. Die austretenden Spinnfäden wiesen je nach lokaler Temperatur und somit Viskosität der Spinnmasse einen Durchmesser von ca. 50 µm auf. Das fließfähige, honigartige Material fiel durch sein Eigengewicht in einen unter dem Druckzylinder befindlichen Spinnschacht mit 2 m Länge. Im Spinnschacht wurden Temperatur und Feuchtigkeit kontrolliert eingestellt. Die Temperatur lag bei 25°C und die Luftfeuchte bei 19%. Beim Auftreffen der Fäden auf den Changiertisch behielten diese nahezu ihre zylindrische Form bei, waren jedoch noch so fließfähig, dass sie an ihren Berührungsflächen miteinander zu Fasegelegen (Vliesen) verklebten.

### 2. Konstitutive und induzierte Interleukin-1β Synthese bei Keratinocyten

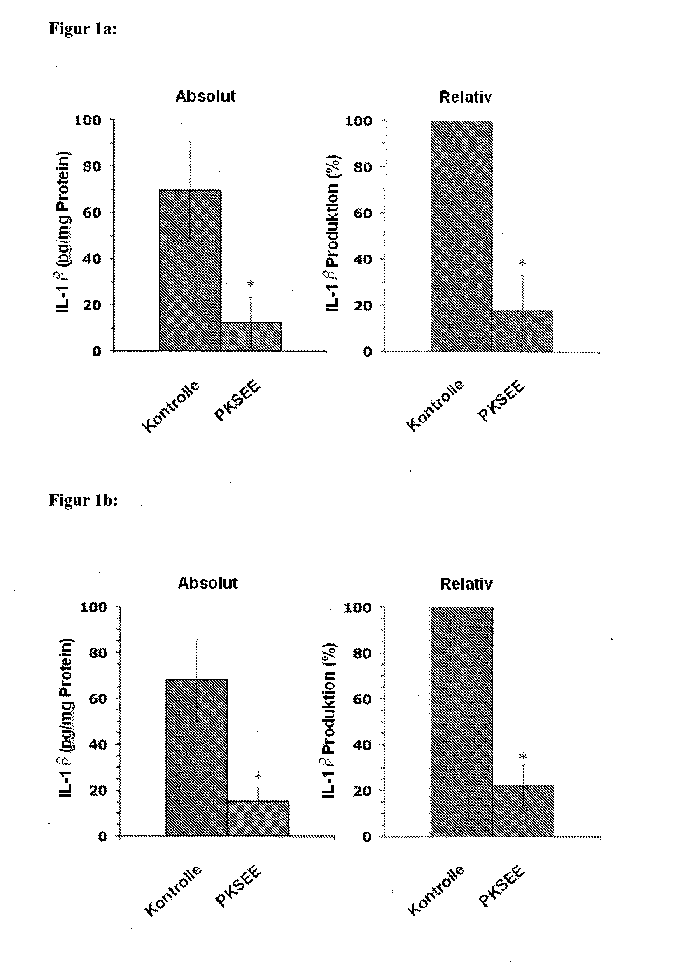

Zur Bestimmung des Einflusses der in Beispiel 1 hergestellten Fasermatrix aus Polyhydroxykieselsäureethylester (in den Figuren als PKSEE abgekürzt) auf die konstitutive Interleukin-1β Synthese bei Keratinocyten wurden 6-Well Zellkulturplatten mit 200.000 Keratinocyten und 3 ml Wachstumsmedium in jedem Well eingesetzt. Die Zellkulturplatten wurden dann mit geeigneten Kunststoffeinhänger bestückt. Im Falle der Testung der Polyhydroxykieselsäureethylester-Verbindung wurden jeweils 1 cm² einer Polyhydroxykieselsäureethylester-Fasermatrix in die Einhänge eingebracht und mit 1 ml Medium überschichtet. In Kontrollen wurde die Einhänge mit 1 ml Medium ergänzt. Der Inhalt der Kunststoffeinhänge ist durch eine Membran von dem Medium der Zellkultur getrennt. Aufgrund der Membrandurchlässigkeit ist jedoch ein Stoffaustausch von gelösten Substanzen zwischen dem Inhalt der Einhänge und dem Zellkulturmedium möglich. Die Kulturplatten wurden für 2 bis 4 Tage kultiviert. Die absolute und relative Menge an Interleukin-1β im Zellkulturüberstand wurde danach mit einem ELISA Assay [Human IL-1 beta ELISA kit von R&D Systems] bestimmt. Figur 1a zeigt eine starke und signifikante Reduktion der konstitutiven Interleukin-1β Synthese nach 72 Stunden Kultur [Student's t-Test].

Ein weiterer Versuch wurde analog durchgeführt mit dem einzigen Unterschied, dass die Keratinocyten zu Beginn für 24 Stunden durch Zugabe von IL-β [500 Units/ml] aktiviert wurden. Anschließend wurden in den Kulturen durch mehrfaches Waschen das exogen zugesetzte IL-1β entfernt und die Kulturen wurden für weitere 24 Stunden im normalen Wachstumsmedium kultiviert. Figur 1b zeigt eine im Vergleich zu unbehandelten Kulturen starke und signifikante [Student's t-Test] Reduktion der induzierten Interleukin 1β Synthese der mit dem erfindungsgemäßen Material behandelten Keratinocytenkulturen.

### 3. Konstitutive und induzierte Interleukin-6 Synthese bei Keratinocyten, Endothelzellen und Fibroblasten

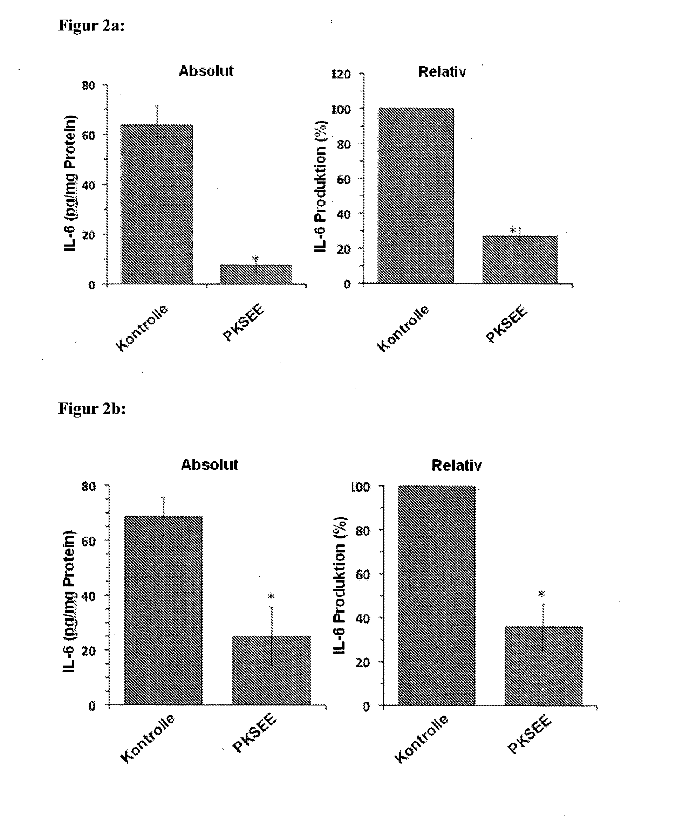

Gleiche Versuche wie bei Bsp. 2 beschrieben wurden durchgeführt mit dem Unterschied, dass anstatt Interleukin-1β die absolute und relative Menge von Interleukin-6 im Zellkulturüberstand mit einem ELISA Assay [Human IL-6 ELISA kit von R&D Systems] bestimmt wurde. Figur 2a zeigt eine starke und signifikante [Student's t-Test] Reduktion der konstitutiven Interleukin 6 Synthese bei Keratinocyten nach 72 Stunden Kultur.

Ein weiterer Versuch wurde analog durchgeführt mit dem einzigen Unterschied, dass die Keratinocyten zu Beginn für 24 Stunden durch Zugabe von IL-1β [500 Units/ml] aktiviert wurden. Anschließend wurden in den Kulturen durch mehrfaches Waschen das exogen zugesetzte IL-1β entfernt und die Kulturen wurden für weitere 24 Stunden im normalen Wachstumsmedium kultiviert. Figur 2b zeigt eine im Vergleich zu unbehandelten Kulturen starke und signifikante [Student's t-Test] Reduktion der durch das IL-1β induzierten Interleukin-6 Synthese der mit dem erfindungsgemäßen Material behandelten Keratinocytenkulturen.

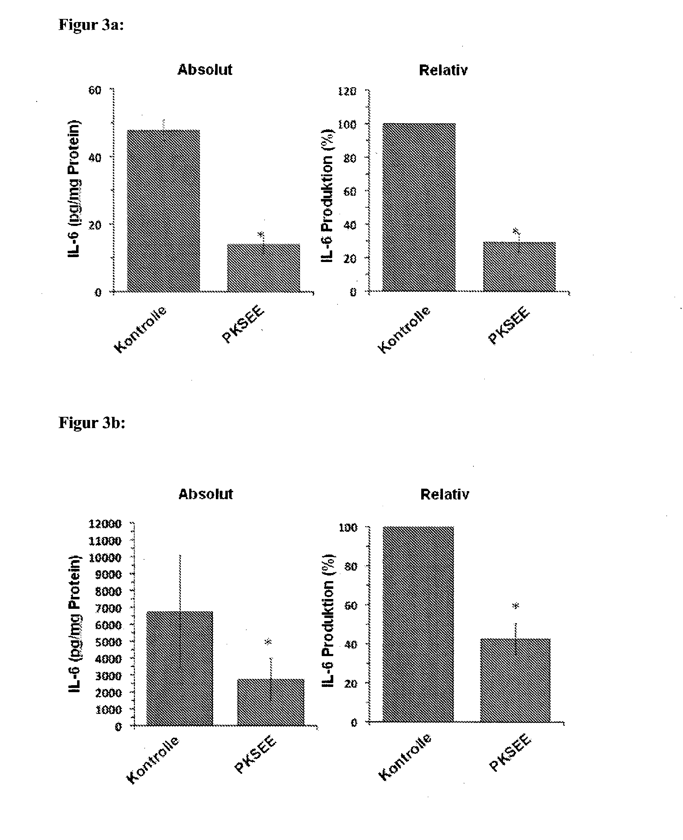

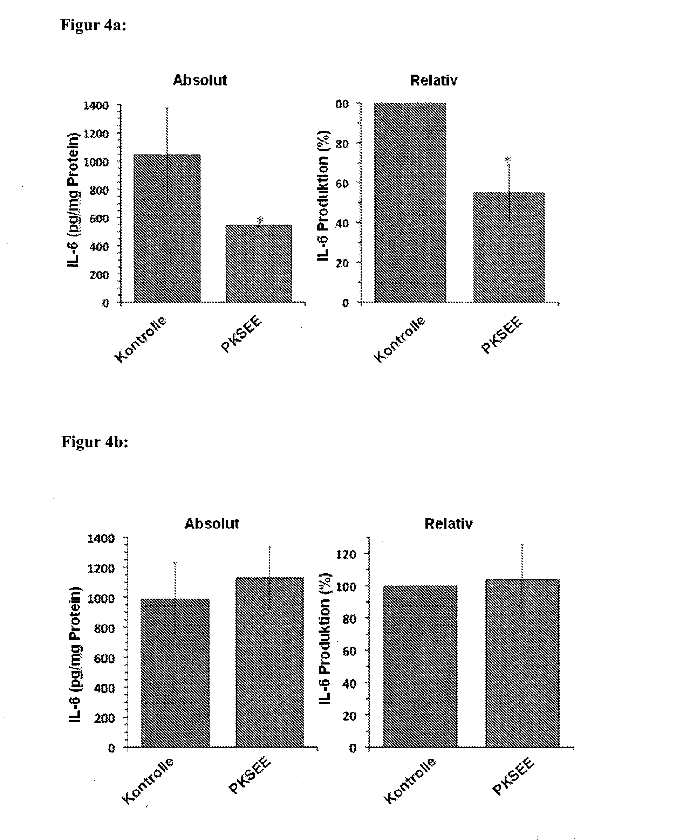

Gleiche Versuche wurden für Endothelzellen und Fibroblasten anstatt Keratinocyten durchgeführt. Figur 3a zeigt eine starke und signifikante [Student's t-Test] Reduktion der konstitutiven Interleukin-6 Synthese bei Endothelzellen nach 72 Stunden. Figur 3b zeigt eine starke und signifikante [Student's t-Test] Reduktion der IL-1β-induzierten Interleukin-6 Synthese bei Endothelzellen nach 72 Stunden. Figur 4a zeigt eine starke und signifikante [Student's t-Test] Reduktion der konstitutiven Interleukin-6 Synthese bei Fibroblasten nach 72 Stunden. Figur 4b zeigt keine signifikante [Student's t-Test] Reduktion der IL-1β-induzierten Interleukin-6 Synthese bei Fibroblasten.

### 4. Konstitutive und induzierte Interleukin-8 Synthese bei Keratinocyten, Endothelzellen und Fibroblasten

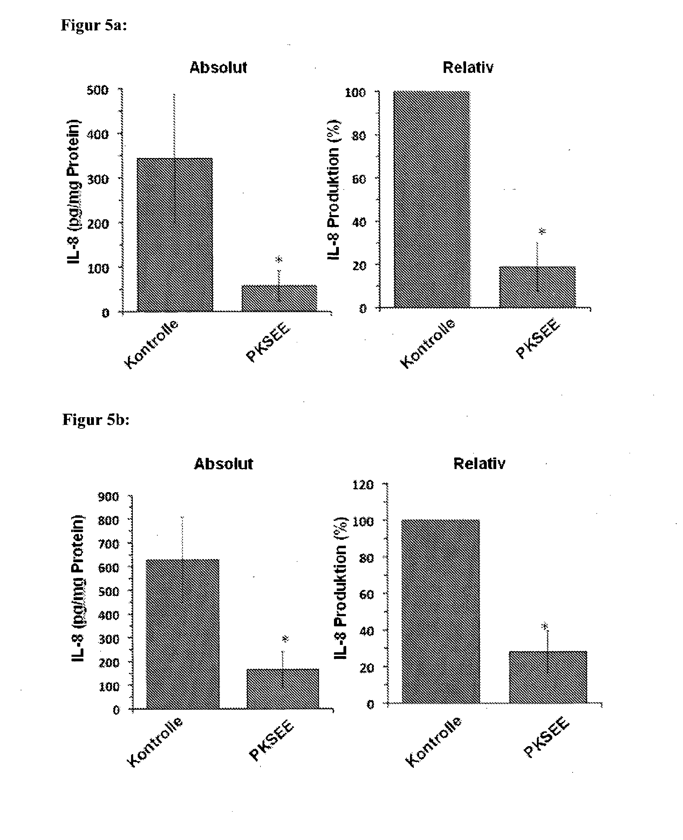

Gleiche Versuche wie bei Bsp. 2 beschrieben wurden durchgeführt mit dem Unterschied, dass anstatt Interleukin-1β die absolute und relative Menge an Interleukin-8 in den Zellkulturüberständen mit einem ELISA Assay [Human IL-8 ELISA kit von R&D Systems] bestimmt wurde. Figur 5a zeigt eine starke und signifikante [Student's t-Test] Reduktion der konstitutiven Interleukin-8 Synthese bei Keratinocyten nach 72 Stunden.

Weitere Versuche wurde analog durchgeführt mit dem einzigen Unterschied, dass die Keratinocyten zu Beginn für 24 Stunden durch Zugabe von IL-β [500 Units/ml] aktiviert wurden. Anschließend wurden in den Kulturen durch mehrfaches Waschen das exogen zugesetzte IL-1β entfernt und die Kulturen wurden für weitere 24 Stunden im normalen Wachstumsmedium kultiviert. Figur 5b zeigt eine im Vergleich zu unbehandelten Kulturen starke und signifikante [Student's t-Test] Reduktion der durch das IL-1β induzierten Interleukin-8 Synthese der mit dem erfindungsgemäßen Material behandelten Keratinocytenkulturen.

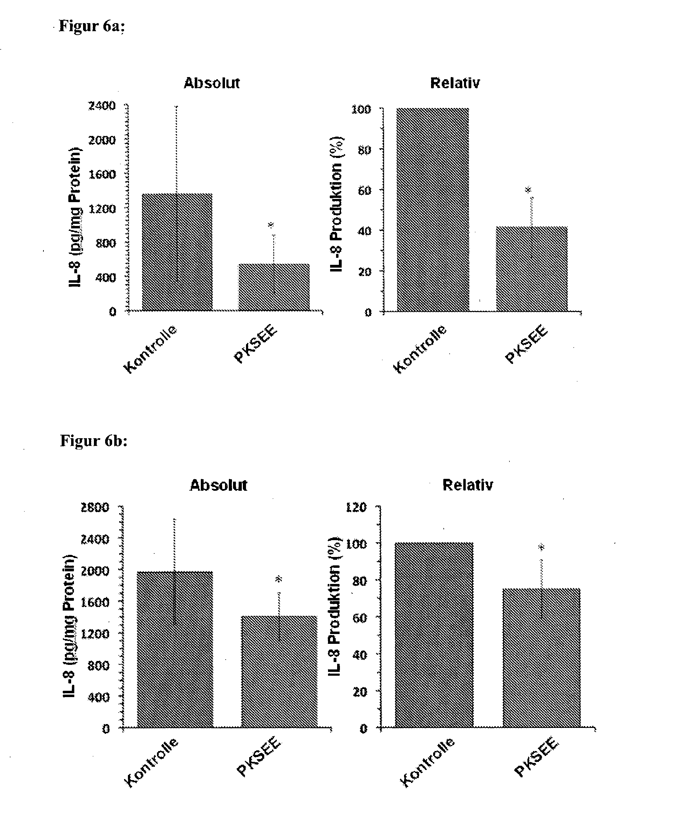

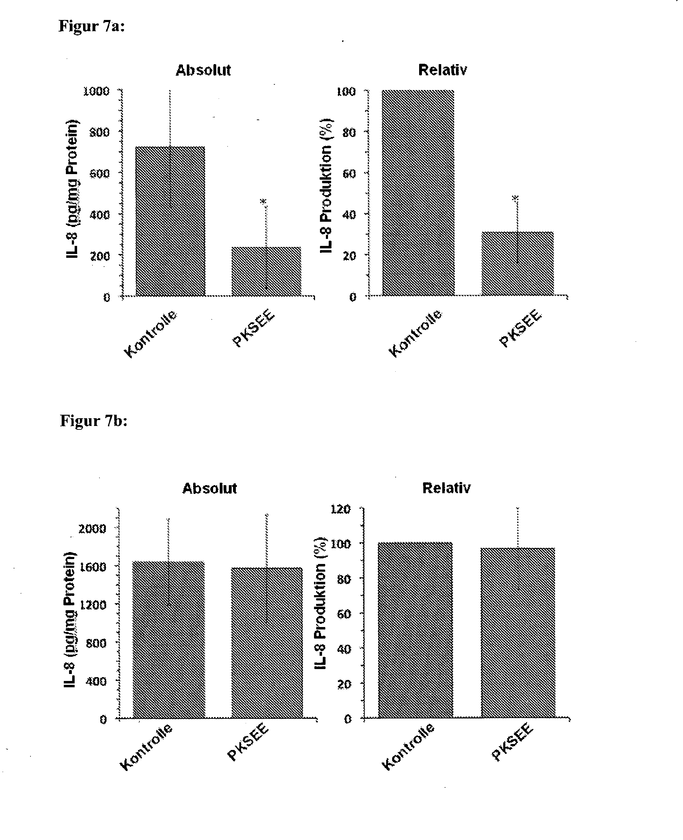

Die gleichen Versuche wurden mit Endothelzellen und Fibroblasten anstatt Keratinocyten durchgeführt. Figur 6a zeigt eine starke und signifikante Reduktion der konstitutiven Interleukin-8 Synthese bei Endothelzellen nach 72 Stunden Kultur. Figur 6b zeigt eine signifikante [Student's t-Test] Reduktion der induzierten Interleukin-8 Synthese bei Endothelzellen nach 72 Stunden. Figur 7a zeigt eine starke und signifikante [Student's t-Test] Reduktion der konstitutiven Interleukin-8 Synthese bei Fibroblasten nach 72 Stunden. Figur 7b zeigt keine signifikante Reduktion der induzierten Interleukin 8 Synthese bei Fibroblasten.

Figuren 1a, 2a, 3a, 4a, 5a, 6a und 7a: *; p<0.05 im Vergleich zur Kontrolle; n = 9; Figuren 1b, 2b, 3b, 4b, 5b, 6b und 7b: *; p<0.05 im Vergleich zur Kontrolle; n = 12; die statistische Auswertung erfolgten mit dem Student's t-Test.

## Patentansprüche

1. Siliciumhaltiges, biologisch degradierbares Material zur Verwendung in der Prophylaxe und/oder zur Behandlung von Krankheiten, die mit einer erhöhten Interleukin-1β und/oder Interleukin-6 und/oder Interleukin-8 Aktivität einhergehen und/oder Krankheiten bei denen eine Reduktion der Aktivität von Interleukin-1β und/oder Interleukin-6 und/oder Interleukin-8 förderlich für den Heilungsprozess ist,
wobei das siliciumhaltige, biologisch degradierbare Material eine Polyhydroxykieselsäureethylester-Verbindung der allgemeinen Formel H[OSi₈O₁₂(OH)ₓ(OC₂H₅)₆₋ₓ]ₙOH ist, bei der x für 2 bis 5 steht und n > 1 ist,
wobei die Krankheiten ausgewählt sind aus der Gruppe von:
Autoimmunerkrankungen, Enteritis, Kolitis, Gastritis, Arthritis, Myokarditis, Dermatitis, Otitis, Pneumonitis, Schocklunge, Blutgerinnungsstörungen, entzündliche Knochen- und Gelenkerkrankungen, Rheumatoide Arthrithis, Sepsis, Septischer Schock, Folgeerscheinungen nach Transplantationen, akute und chronische Entzündungen, entzündliche Darmerkrankung (Bowel disease), Graft-versus-Host-Krankheit, Schock, Schlaganfall, akutes respiratorisches Syndrom (ARDS), Psoriasis, Restenose, AIDS, Kachexie, Schädel-Hirn-Trauma Allergie, parasitäre Infektion, allergische Rhinitis, allergisches Asthma, Multiple Sklerose, Systemischer Lupus erythematodes, Graft-versus-Host-Krankheit, Transplantatabstoßung, Asthma und chronisch obstruktiver Lungenerkrankung, chronische Gastritis, akute und chronische Entzündungen der Haut, Allergien der Haut, parasitäre Infektion der Haut, atopische Dermatitis insbesondere Neurodermitis, Dermatomyositis, Pemphigus vulgaris und/oder andere lokale und systemische Infektionen und/oder akute und chronische Entzündungssituationen,
mit der Maßgabe das Wunddefekte, wie chronische diabetisch-neuropathische Ulcus, Ulcus cruris, Dekubituswunden, sekundär heilende infizierte Wunden, reizlose, primär heilende Wunden, wie insbesondere ablative Riß- oder Schürfwunden ausgenommen sind.

2. Siliciumhaltiges, biologisch degradierbares Material zur Verwendung nach Anspruch 1, wobei das Material in Form einer Faser, einer Fasermatrix, als Pulver, als flüssige Formulierung, als Monolith und/oder als Beschichtung vorliegt.

3. Siliciumhaltiges, biologisch degradierbares Material zur Verwendung nach Anspruch 2, hergestellt durch:
a) mindestens einer Hydrolyse-Kondensationsreaktion von Tetraethoxysilan
b) Eindampfen zur Erzeugung einer einphasigen Lösung vorzugsweise bei einer gleichzeitigen schonenden Durchmischung des Reaktionssystems,
c) Abkühlung der einphasigen Lösung und
d) Reifung zur Erzeugung des Kieselsol-Materials
e) Ziehen von Fäden von dem Kieselsol-Material zur Generierung einer Faser bzw.
einer Fasermatrix und/oder
Trocknen, insbesondere Sprüh- oder Gefriertrocknen des Kieselsol-Materials zur Generierung eines Pulvers und ggfs. Lösen des Pulvers in einem Lösungsmittel zur Generierung einer flüssigen Formulierung und/oder
Beschichten eines mit dem siliciumhaltigen, biologisch degradierbaren Material zu beschichtendem Gegenstandes mit dem Kieselsol-Material, und/oder Gießen des Kieselsol-Materials in eine Form zur Generierung eines Monoliths.

4. Siliciumhaltiges, biologisch degradierbares Material zur Verwendung nach Anspruch 3,
wobei das Tetraethoxysilan in Schritt a) bei einem anfänglichen pH-Wert von 0 bis ≤ 7, gegebenenfalls in Gegenwart eines wasserlöslichen Lösungsmittels, vorzugsweise Ethanol, bei einer Temperatur von 0°C bis 80°C sauer katalysiert wird und
in Schritt b) durch Eindampfen eine einphasige Lösung bis zu einer Viskosität im Bereich von 0,5 bis 2 Pa • s bei einer Scherrate von 10 s⁻¹ bei 4 °C durchgeführt wird.

5. Siliciumhaltiges, biologisch degradierbares Material zur Verwendung nach Anspruch 4, wobei die saure Katalyse in Schritt a) mit salpetersaurem H₂O in einem molaren Verhältnis zur Silicium-Verbindung im Bereich 1:1,7 bis 1:1,9, bevorzugt im Bereich von 1:1,7 bis 1:1,8 durchgeführt wird.

## Claims

1. Silicon-containing, biodegradable material for use in preventing and/or for treating diseases that are associated with increased interleukin-1β and/or interleukin-6 and/or interleukin-8 activity and/or diseases in which a reduction in the activity of Interleukin-1β and/or interleukin-6 and/or interleukin-8 is beneficial for the healing process,
wherein the silicon-containing, biodegradable material is a polyhydroxysilicic acid ethyl ester compound of the general formula H[OSi₈O₁₂-(OH)ₓ(OC₂H₅)₆₋ₓ]ₙOH, in which x represents 2 to 5 and n > 1,
wherein the diseases are selected from the group of:
autoimmune diseases, enteritis, colitis, gastritis, arthritis, myocarditis, dermatitis, otitis, pneumonitis, shock lung, blood clotting disorders, inflammatory bone and joint diseases, rheumatoid arthritis, sepsis, septic shock, post-transplantation sequelae, acute and chronic inflammations, inflammatory bowel disease, graft-versus-host disease, shock, stroke, acute respiratory syndrome (ARDS), psoriasis, restenosis, AIDS, cachexia, cerebrocranial trauma, allergy, parasitic infection, allergic rhinitis, allergic asthma, multiple sclerosis, systemic lupus erythematosus, graft-versus-host disease, transplant rejection, asthma and chronic obstructive pulmonary disease, chronic gastritis, acute and chronic inflammations of the skin, skin allergies, parasitic skin infection, atopic dermatitis in particular neurodermatitis, dermatomyositis, pemphigus vulgaris and/or other local and systemic infections and/or acute and chronic inflammatory situations,
with the proviso that wound defects, such as chronic diabetic-neuropathic ulcer, chronic leg ulcer, bedsores, secondary-healing infected wounds, nonirritating, primary-healing wounds, such as in particular ablative lacerations or abrasions, are excluded.

2. Silicon-containing, biodegradable material for use according to Claim 1, wherein the material is in the form of a fibre, a fibre matrix, as powder, as liquid formulation, as monolith and/or as coating.

3. Silicon-containing, biodegradable material for use according to Claim 2, produced by:
a) at least one hydrolysis-condensation reaction of tetraethoxysilane
b) evaporation for producing a single-phase solution preferably with simultaneous gentle mixing of the reaction system,
c) cooling of the single-phase solution and
d) maturation for production of the silica sol material
e) drawing of threads from the silica sol material to generate a fibre or
a fibre matrix and/or
drying, in particular spray drying or freeze-drying of the silica sol material to generate a powder and optionally dissolving the powder in a solvent to generate a liquid formulation and/or
coating the silica sol material on an object that is to be coated with the silicon-containing, biodegradable material, and/or
casting the silica sol material in a mould to generate a monolith.

4. Silicon-containing, biodegradable material for use according to Claim 3, wherein the tetraethoxysilane is acid-catalysed in step a) at an initial pH from 0 to ≤ 7, optionally in the presence of a water-soluble solvent, preferably ethanol, at a temperature from 0°C to 80°C and
in step b), a single-phase solution is evaporated to a viscosity in the range from 0.5 to 2 Pa • s at a shear rate of 10 s⁻¹ at 4°C.

5. Silicon-containing, biodegradable material for use according to Claim 4, wherein the acid catalysis in step a) is carried out with aqueous solution of nitric acid in a molar ratio to the silicon compound in the range 1:1.7 to 1:1.9, preferably in the range from 1:1.7 to 1:1.8.

## Revendications

1. Matériau biodégradable contenant du silicium, pour utilisation en prophylaxie et/ou traitement de maladies qui sont dues à une activité accrue d'interleukine-1β et/ou d'interleukine-6 et/ou d'interleukine-8, et/ou de maladies dans lesquelles une réduction de l'activité de l'interleukine-1β et/ou de l'interleukine-6 et/ou de l'interleukine-8 est avantageuse pour le processus de guérison,
le matériau biodégradable contenant du silicium, étant un composé ester éthylique de l'acide polyhydroxysilicique de formule générale H[OSi₈O₁₂(OH)ₓ(OC₂H₅)₆₋ₓ]ₙOH, dans laquelle x vaut 2 à 5, et n > 1,
les maladies étant choisies dans le groupe consistant en
les maladies auto-immunes, l'entérite, la colite, la gastrite, l'arthrite, la myocardite, la dermatite, l'otite, la pneumonie, le poumon de choc, les troubles de la coagulation sanguine, les maladies osseuses et articulaires inflammatoires, la polyarthrite rhumatoïde, la sepsie, le choc septique, les séquelles après transplantations, les inflammations aiguës et chroniques, l'affection abdominale inflammatoire (bowel disease), la réaction de greffe contre hôte, le choc, l'accident vasculaire cérébral, le syndrome de détresse respiratoire de l'adulte (SDRA), le psoriasis, la resténose, le SIDA, la cachexie, la commotion cérébrale, l'allergie, l'infection parasitaire, la rhinite allergique, l'asthme allergique, la sclérose en plaques, le lupus érythémateux disséminé, la réaction greffe contre hôte, le rejet des transplantations, l'asthme et la broncho-pneumopathie chronique obstructive, la gastrite chronique, les inflammations aiguës et chroniques de la peau, les allergies de la peau, l'infection parasitaire de la peau, la dermatite atopique, notamment la neurodermite, la dermatomyosite, le pemphigus vulgaire et/ou d'autres infections locales et systémiques, et/ou les situations inflammatoires aiguës et chroniques,
à la condition d'exclure les déficiences des plaies telles que l'ulcère diabétique-neuropathique chronique, l'ulcère de la jambe, les escarres de décubitus, les plaies infectées à cicatrisation secondaire, les plaies non irritées à cicatrisation primaire telles en particulier que les lacérations ou abrasions ablatives.

2. Matériau biodégradable contenant du silicium, pour l'utilisation selon la revendication 1, le matériau se présentant sous forme d'une fibre, d'une matrice fibreuse, sous forme d'une poudre, sous forme d'une formulation liquide, sous forme d'un monolithe et/ou sous forme d'un revêtement.

3. Matériau biodégradable contenant du silicium, pour l'utilisation selon la revendication 2, fabriqué par :
a) au moins une réaction de condensation par hydrolyse de tétraéthoxysilane,
b) évaporation pour produire une solution monophasique, de préférence en présence d'un mélange intime ménagé simultané du système réactionnel,
c) refroidissement de la solution monophasique, et
d) maturation pour la production du matériau sol de silice,
e) tirage de fils du matériau sol de silice pour générer une fibre ou une matrice fibreuse, et/ou
séchage, en particulier séchage par atomisation ou lyophilisation, du matériau sol de silice, pour produire une poudre, et éventuellement dissolution de la poudre dans un solvant pour produire une formulation liquide, et/ou
application du matériau sol de silice sur un objet devant être revêtu du matériau biodégradable contenant du silicium, et/ou
coulée du matériau sol de silice sous une forme permettant la production d'un monolithe.

4. Matériau biodégradable contenant du silicium, pour l'utilisation selon la revendication 3,
dans lequel le tétraéthoxysilane de l'étape a) est soumis à une catalyse acide à une température de 0 à 80°C, à un pH initial de 0 à ≤ 7, éventuellement en présence d'un solvant soluble dans l'eau, de préférence l'éthanol, et
dans l'étape b), on procède par évaporation à une dissolution monophasique jusqu'à une viscosité comprise dans la plage de 0,5 à 2 Pa·s pour une vitesse de cisaillement de 10 s⁻¹ à 4°C.

5. Matériau biodégradable contenant du silicium, pour l'utilisation selon la revendication 4, dans lequel la catalyse acide de l'étape a) est mise en oeuvre avec du H₂O acidifié par de l'acide nitrique pour un rapport en moles au composé du silicium compris dans la plage de 1:1,7 à 1:1,9, de préférence dans la plage de 1:1,7 à 1:1,8.
